# EUROPEAN PATENT APPLICATION

(11) **EP 1 829 615 A1**
(43) Date of publication of application: **05.09.2007**
(21) Application number: 06714067.3
(22) Date of filing: 20.02.2006
(51) Int. Cl.: B03C 3/41, A61L 9/16, B03C 3/02, B03C 3/47

(54) **ELECTRIC DUST-COLLECTING UNIT**

(30) Priority: 21.02.2005 JP 2005043713; 08.03.2005 JP 2005063305; 08.03.2005 JP 2005063306
(71) Applicant: MATSUSHITA ELECTRIC INDUSTRIAL CO., LTD., Osaka 571-8501 (JP)
(72) Inventor: KAI, Tooru c/o Matsushita Electric Industrial Co., Ltd., Chuo-ku, Osaka-shi, Osaka 540-6319 (JP); IWAHASHI, Hiromu c/o Matsushita Electric Industrial Co., Ltd., Chuo-ku, Osaka-shi, Osaka 540-6319 (JP)
(74) Representative: Pautex Schneider, Nicole Véronique
(86) International application number: PCT/JP2006/302926
(87) International publication number: WO 2006/088174

(57) **Abstract**

Provided is an electric dust-collecting unit having an improved dust-collecting performance and a high potential for reduction in thickness. The unit comprises a plurality of needle-shaped electrodes (201) having pointed ends (205) oriented in a direction of airflow, parallel electrodes (203) disposed in parallel to needle-shaped electrodes (201), and a perforated electrode (204) disposed at a downstream side of needle-shaped electrodes (201) in a position to confront pointed ends (205). Parallel electrodes (203) and perforated electrode (204) are arranged in a shape of a letter U in a manner to surround needle-shaped electrodes (201). This achieves electrical discharges to spread widely from needle-shaped electrodes (201) to the confronting electrodes and improves the dust-collecting performance. It also allows to bring needle-shaped electrodes (201) closely to perforated electrode (204) so as to provide the electric dust-collecting unit with a reduced thickness.

## Description

### TECHNICAL FIELD

The present invention relates to an electric dust-collecting unit placed in a space of air stream of such a product as an air conditioner for removing dust and the like contained in the air being conditioned.

### BACKGROUND ART

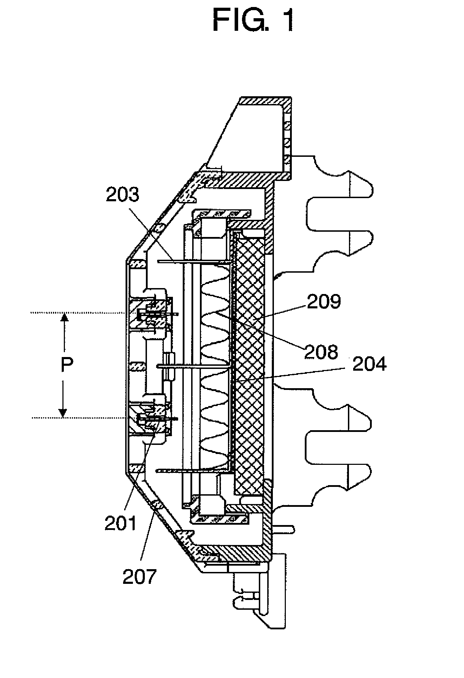

Typical electric dust-collecting unit of the kind hitherto known is provided with discharge electrodes of a needle shape (for example, refer to Japanese Patent Unexamined Publication, No. 2003-10731, Fig. 1).

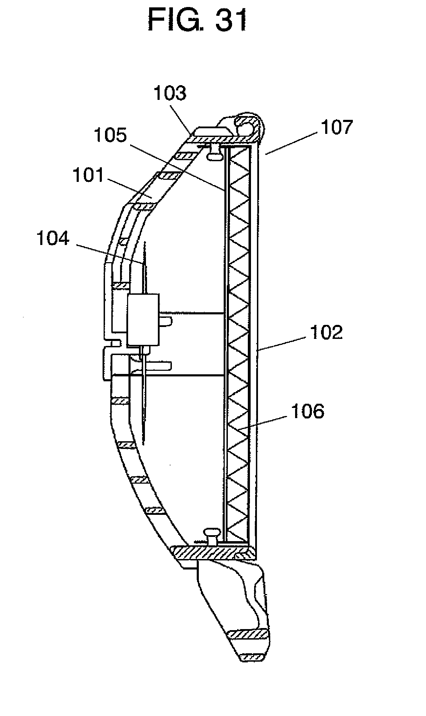

### Description is provided hereinafter of the electric dust-collecting unit with reference to Fig. 31.

Discharge electrodes 104 of the needle shape are disposed at the side of air intake opening 101 of filter frame 103 that has both air intake opening 101 and outlet opening 102, as shown in Fig. 31. There is also ground electrode 105 of a ventilative form such as a latticed or punched metal plate, which is disposed over the outlet opening 102 of filter frame 103 in a manner to confront discharge electrodes 104. Filter 106 is placed in the outflow side of ground electrode 105 to make up filter assembly 107. The conventional electric dust-collecting unit of this type has discharge electrodes 104 so oriented that their pointed ends are generally in parallel to the plane of ground electrode 105, which does not yield a sufficient degree of dust-collecting performance, and that this structure is not suitable for making the electric dust-collecting unit small in thickness.

### SUMMARY OF THE INVENTION

The present invention is to overcome the problems discussed above, and to provide an electric dust-collecting unit having a high dust-collecting performance as well as a capability of reducing the thickness

The electric dust-collecting unit of the present invention comprises a plurality of needle-shaped electrodes having their pointed ends oriented in a direction of airflow, and parallel electrodes disposed in parallel to the needle-shaped electrodes. Provided further is a perforated electrode at the downstream side of the needle-shaped electrodes in a position to confront their pointed ends, so that the parallel electrodes and the perforated electrode are arranged in a shape of a letter U in a manner to surround each of the needle-shaped electrodes. This structure helps electrical discharges to spread uniformly in many directions from the pointed ends of the needle-shaped electrodes to the confronting electrodes, and it can thereby improve the dust-collecting performance. The structure also allows such an arrangement that the needle-shaped electrodes are disposed more closely to the perforated electrode, so as to reduce the thickness of the electric dust-collecting unit.

In addition, the electric dust-collecting unit is so constructed that a distance from the pointed ends of the needle-shaped electrodes to the parallel electrodes is equal to a distance to the perforated electrode. Since the pointed ends of the needle-shaped electrodes are arranged at the equally uniform distance to both the parallel electrodes and the perforated electrode, this arrangement can reduce variations in discharge intensity as well as directions of the electrical discharges. Moreover, since the parallel electrodes and the perforated electrode are arranged to form the shape of generally the letter U, the electrical discharges occur uniformly in the spaces formed therebetween, thereby improving the dust-collecting performance and increasing the dust-collecting efficiency.

This electric dust-collecting unit is so constructed that the distance of electrical discharges from the pointed ends of the needle-shaped electrodes to the parallel electrodes is set to be 6mm to 15mm, and an applied voltage to -4kV to -8kV according to these discharge electrodes. These values can provide the optimum conditions for efficient discharges, reduce variations in dust-collecting efficiency, and ensure a high rate of the dust collection.

Furthermore, the electric dust-collecting unit has the needle-shaped electrodes formed on a needle-shaped electrode plate at intervals of 8mm to 30mm. If the intervals are too small between the pointed ends of the adjoining needle-shaped electrodes, an excessively large amount of current flows especially when they carry a high voltage. It is therefore necessary to increase the intervals. On the other hand, the current flow tends to diminish as the intervals increase when the voltage is low, and it hence becomes necessary to bring the intervals closer. It is for this reason to provide the intervals of 8mm to 30mm between the needle-shaped electrodes in order to satisfy both of these conditions. This can suppresses undesired electrical discharges attributable to interference between the pointed ends of the needle-shaped electrodes, and produce the electrical discharges uniformly to the parallel electrodes and the perforated electrode to achieve an improvement of the dust-collecting efficiency.

The electric dust-collecting unit has the needle-shaped electrode plate fabricated of a sheet metal into a shape comprising the needle-shaped electrodes of an acute angle at their pointed ends and a base portion widened to reinforce them. The needle-shaped electrodes are secured firmly to a frame made of a resin material at the widened base portion for reinforcement, so that the needle-shaped electrodes are held to maintain their pointed ends at predetermined positions. Since the needle-shaped electrodes can be formed of the sheet metal, it is possible to eliminate a welding process to attach discharging needles individually so as to achieve a cost reduction.

Moreover, the electric dust-collecting unit is provided with a plurality of needle-shaped electrode plates, each having a number of the needle-shaped electrodes. The needle-shaped electrode plates are arranged in parallel to one another at regular intervals, and another plurality of tabular parallel electrodes are disposed in parallel to these needle-shaped electrode plates at regular intervals.

The electric dust-collecting unit is also provided with a tabular perforated electrode which supports the parallel electrodes, and that the electric dust-collecting unit is adaptable to an increase or decrease in an area available for installation by adjusting numbers of the needle-shaped electrode plates and the parallel electrodes as well as dimensions of the perforated electrode. A single dust collector unit consisting of a parallel electrode, a needle-shaped electrode plate and a perforated electrode may be prepared as a basic unit, so as to increase versatility for adapting it to various types and shapes of electric dust-collecting units. This can also help make the parallel electrodes and the needle-shaped electrode plates as components for common use.

In addition, the perforated electrode can be enlarged to any integral multiple of the basic unit by simply increasing an area size of the components. This enables the electric dust-collecting unit to be designed according to a configuration and dimensions of an airflow area of any variety of air conditioners, for example. In other words, this can standardize the components and achieve a cost reduction.

The electric dust-collecting unit is also provided with a dust-collecting filter disposed at the downstream side in a manner to contact with the perforated electrode. The dust-collecting filter collects dust statically charged by electrical discharges generated from the pointed ends of the needle-shaped electrodes when a high voltage is applied thereto, thereby achieving an improvement of the dust-collecting efficiency.

The electric dust-collecting unit is additionally provided with a deodorizing filter disposed at the downstream side in a manner to contact with the perforated electrode. The deodorizing filter collects odorous components along with the dust statically charged by electrical discharges generated from the pointed ends of the needle-shaped electrodes when the high voltage is applied thereto. This structure can hence improve the deodorizing effect in addition to performing the dust-collecting function sufficiently.

In the electric dust-collecting unit, any of the dust-collecting filter and the deodorizing filter disposed at the downstream side of the perforated electrode is composed to have an electrical conductivity. This structure can make the entire surface of the electrically conductive dust-collecting filter or the deodorizing filter in contact with the perforated electrode, and maintain them at the ground potential. This structure can ensure collection of dust and the odorous components included in the dust statically charged by electrical discharges generated from the pointed ends of the needle-shaped electrodes when the high voltage is applied, so as to increase the dust-collecting efficiency as well as the deodorizing efficiency.

The electric dust-collecting unit may be provided with any of dust-collecting filters and deodorizing filters disposed in parallel to one another at the upstream side of the perforated electrode, and at least back surfaces of these dust-collecting filters or the deodorizing filters are kept in contact with the perforated electrode. This structure can maintain the whole filters consisting of the dust-collecting filters or the deodorizing filters at the ground potential, thereby facilitating the entire filters to collect the dust charged statically around the pointed ends of the needle-shaped electrodes as the high voltage is applied. The structure can thus increase the dust-collecting efficiency. Besides, since the side surfaces of the filters are in contact with the parallel electrodes and maintained in continuity to the ground potential, this electric dust-collecting unit can further improve the dust-collecting efficiency.

In the electric dust-collecting unit, any of the dust-collecting filters and the deodorizing filters at the upstream side on of the perforated electrode are made to be electrically conductive, and the dust-collecting filters or the deodorizing filters are so disposed that a distance of their upper surfaces from the pointed ends of the needle-shaped electrodes is equal to the distance from the pointed ends to the parallel electrodes. This eliminates a problem of electrical discharges to concentrate only around the upper surfaces of the dust-collecting filters, so as to facilitate the discharges to spread generally evenly over the dust-collecting filters and the parallel electrodes. The structure can thus collect the statically charged dust instantly with the dust-collecting filters, and improve the dust-collecting efficiency.

The electric dust-collecting unit is provided with a plurality of chemical agents coated separately on each of at least two dust-collecting filters. The chemical agents not easily applicable at once in a form of multiple layer on a filter can instead be applied separately to each of the plurality of filters provided. This structure can hence realize the electric dust-collecting unit as being multi-purpose and multi-functional, which is suitable for various kinds of orders, gases and active components even in a single unit.

The electric dust-collecting unit is provided with any of first dust-collecting filters and first deodorizing filters disposed at the upstream side of the perforated electrode, and any of a second dust-collecting filter and a second deodorizing filter at the downstream side of the perforated electrode. This realizes a double-layer structure of any of the dust-collecting filters and the deodorizing filters at both the upstream side and the downstream side, so as to make arrangement of the filters changeable according to the end use application. This structure can thus improve any of the dust-collecting efficiency and the deodorizing efficiency.

Also, the electric dust-collecting unit is so designed as to be adaptable to an increase or decrease in an area available for installation by adjusting a number of any of the first dust-collecting filters and the first deodorizing filters disposed at the upstream side of the perforated electrode as well as dimensions of any of the second dust-collecting filter and the second deodorizing filter disposed at the downstream side of the perforated electrode. For example, identically configured filters to be disposed to the upstream side of the perforated electrode may be increased in number in order to conform to any shape and size of air conditioners of various types. Since this facilitates common use and standardization of the components, it helps ease design changes of the electric dust-collecting unit.

Still another electric dust-collecting unit of the present invention comprises a storage frame for storing a ground electrode provided with a first filter and a second filter. This storage frame is provided with a holder frame for fixing the second filter and the ground electrode, and a grid frame for fixing a needle-shaped electrode. This structure is so constructed that the grid frame is coupled to the storage frame, but not allowed to contact with the holder frame. This structure can ensure predetermined extents of through-the-air spacing and creepage distances, and maintain the safety even when the structure is made into a thin profile capable of coupling the grid frame closely to the storage frame by placing the holder frame for fixing the ground electrode between the storage frame storing the ground electrode and the grid frame provided with the needle-shaped electrode.

The electric dust-collecting unit also has a groove formed in a base portion of a handle provided on the storage frame so that an edge of the grid frame is fitted into the groove. Since the storage frame has the groove for fitting the edge of the grid frame, the groove can play the role of a guide when the grid frame is assembled to the storage frame. The groove can also prevent the grid frame from coming off after the electric dust-collecting unit is assembled.

Moreover, the electric dust-collecting unit is provided with a hook formed at an edge of the storage frame, and a link hole in the grid frame for engagement with the hook, and a surrounding wall formed on the grid frame in a manner to encircle the link hole. Since the link hole for engagement with the hook is bored through the grid frame in the vicinity of an electrical contact, it provides an air passage that allows dust to enter into the interior of a dust collector carrying the electrical contact, and this will result in a leakage inside the dust collector. A problem of this nature can be averted by the surrounding wall encircling the link hole which prevents dust from entering into the interior of the dust collector. This structure can thus avoid the problem of leakage from taking place inside the dust collector, while also play the role of preventing the grid frame from coming off the storage frame after their engagement.

Furthermore, the electric dust-collecting unit is provided with an opening formed in the holder frame for air flow, and a surrounding rib encircling the entire perimeter of the opening. The holder frame can simultaneously make both positioning and fixing of the ground electrode on which the first filter and the second filter are attached. The surrounding rib provided to encircle the entire perimeter of the opening can improve physical strength of the holder frame. It also maintains the proper amount of creepage distances to ensure a sufficient degree of the electrical safety.

The electric dust-collecting unit is also provided with a reinforcing rib bridging between a pair of ribs in the lengthwise direction to form the opening in the holder frame, and a notch formed by cutting off a portion of the ground electrode up to the close vicinity of the upper surface of the first filter. The reinforcing rib is so constructed that the bottom edge of it abuts to the upper edge of the notch. One reinforcing rib or a plurality of them provided across the opening in the holder frame can prevent such a trouble that the ground electrode carrying the first filter and the second filter shifts upward, thereby obviating abnormal electrical discharges and securing a sufficient degree of the electrical safety.

In this electric dust-collecting unit, the reinforcing rib is constructed into such a shape in cross section that it has a thrust rib protruding vertically, and jut ribs projecting both sides from the top of the thrust rib and extending vertically. This structure reinforces the holder frame which secures the ground electrode carrying the first filter and the second filter. The jut ribs ensure a sufficient extent of the creepage distances so as to prevent leakage from taking place, and improve both electrical and mechanical safety.

Moreover, another electric dust-collecting unit of the present invention has a structure described below in order to reduce the thickness while ensuring the creepage distances.

That is, the another electric dust-collecting unit of the invention further comprises a dust-collector side connecting plane and a raised connecting plane provided on an end surface of a dust collector unit, and a ground-side receiving terminal disposed on the raised connecting plane. The electric dust-collecting unit also comprises a power-supply side connecting plane and a recessed connecting plane provided on an end surface of a power supply unit, and a ground-side supply terminal disposed on the recessed connecting plane. The ground-side receiving terminal and the ground-side supply terminal are connected when the raised connecting plane of the dust collector unit and the recessed connecting plane of the power supply unit are engaged. Both the power supply unit and the dust collector unit are provided with this difference in the connecting planes in their respective end surfaces where they engage with each other. This configuration decreases an area of contact between the dust collector unit and the power supply unit and reduces a resistance of frictional during removal and replacement of the dust collector unit from and to the power supply unit. The structure also averts functioning of a fastening part utilizing elasticity of the grounding components until immediately before the complete engagement, so as to improve the usability, ensure sufficiently large extents of the creepage distances and through-the-air clearances between connecting terminals of the high-voltage side and the low-voltage side, and achieve a reduction in the thickness of the electric dust-collecting unit.

Moreover, the electric dust-collecting unit is provided with a recessed portion in which a high-voltage contact point is positioned in the power-supply side connecting plane of the power supply unit, and a projecting barrier formed on the power-supply side connecting plane. The projecting barrier is located between the high-voltage contact point and a grounding contact point. The structure enabling the projecting barrier to be located between the high-voltage contact point and the grounding contact point can ensure the creepage distances sufficiently between the high voltage area and the grounding area.

In the electric dust-collecting unit, this projecting barrier is raised up to a height of the power-supply side connecting plane of the power supply unit. The projecting barrier serves as a guide for sliding movement of the dust collector unit during removal and replacement, and improves usability of the dust collector unit.

Furthermore, the electric dust-collecting unit comprises a support portion provided on one end surface of the dust collector unit opposite the end surface where it is engaged with the power supply unit, and a receptacle for accepting and retaining the support portion. In this structure, the receptacle is mounted to radiator fins of an air conditioner. If the dust collector unit has a cantilevered structure retained only by the power supply unit, the one side opposite the other side where it is retained by the power supply unit tends to tilt when it is engaged to the power supply unit attached to, for instance, an air conditioner. The support portion provided on the one side of the dust collector unit is inserted into the receptacle to prevent the dust collector unit from tilting. This structure also averts the radiator fins from being deformed and damaged, avoids reduction in performance by maintaining the air passage vertically, and simplifies the structure for installation of the electric dust-collecting unit.

Moreover, the electric dust-collecting unit comprises a catch provided in the bottom surface of the receptacle, so as to let the catch hold a projection formed on the support portion. Both sides of the dust collector unit are reliably retained since the catch provided in the receptacle holds the projection formed on the support portion. This structure substantially improves manipulability when removing and replacing the dust collector unit.

In this electric dust-collecting unit, the handle on the dust collector unit is positioned close to the one side where it is engaged with the power supply unit. Since the handle is provided in the position closer to the power supply unit side, where a resistance against removal and replacement of the dust collector unit is larger, it can ease the removal and replacement of the dust collector unit and improve the manipulability.

### BRIEF DESCRIPTION OF DRAWINGS

Fig. 1 is a sectional view showing a structure of an electric dust-collecting unit according to a first exemplary embodiment of the present invention;
Fig. 2 is a sectional view showing a structure of electrodes of the electric dust-collecting unit according to the first exemplary embodiment of the present invention;
Fig. 3A is a characteristic chart showing a relation between distance of electrical discharge and efficiency of the electric dust-collecting unit according to the first exemplary embodiment of the present invention;
Fig. 3B is a characteristic chart showing a relation between the distance of electrical discharge and high voltage output of the electric dust-collecting unit according to the first exemplary embodiment of the present invention;
Fig. 4 is a side view of a needle-shaped electrode plate showing spatial intervals of needle-shaped electrodes of the electric dust-collecting unit according to the first exemplary embodiment of the present invention;
Fig. 5 is a side view showing a configuration of the needle-shaped electrode of the electric dust-collecting unit according to the first exemplary embodiment of the present invention;
Fig. 6 is a characteristic chart showing a relation between intervals of the needle-shaped electrodes and high voltage output of the electric dust-collecting unit according to the first exemplary embodiment of the present invention;
Fig. 7 is a perspective view showing a relation between parallel electrodes and needle-shaped electrodes of the electric dust-collecting unit according to the first exemplary embodiment of the present invention;
Fig. 8A is a sectional view showing a relation between electrodes and a dust-collecting filter of an electric dust-collecting unit according to a second exemplary embodiment of the present invention;
Fig. 8B is a sectional view showing a relation between the electrodes and a deodorizing filter of the electric dust-collecting unit according to the second exemplary embodiment of the present invention;
Fig. 9A is a sectional view showing a relation between electrodes and a dust-collecting filter of an electric dust-collecting unit according to a third exemplary embodiment of the present invention;
Fig. 9B is a sectional view showing a relation between the electrodes and a deodorizing filter of the electric dust-collecting unit according to the third exemplary embodiment of the present invention;
Fig. 10A is a sectional view showing a relation between electrodes and a dust-collecting filter of an electric dust-collecting unit according to a fourth exemplary embodiment of the present invention;
Fig. 10B is a sectional view showing a relation between the electrodes and a deodorizing filter of the electric dust-collecting unit according to the fourth exemplary embodiment of the present invention;
Fig. 11A is a sectional view showing a relation between electrodes and a dust-collecting filter of an electric dust-collecting unit according to a fifth exemplary embodiment of the present invention;
Fig. 11B is a sectional view showing a relation between the electrodes and a deodorizing filter of the electric dust-collecting unit according to the fifth exemplary embodiment of the present invention;
Fig. 12A is a sectional view showing a relation between electrodes and a dust-collecting filter of an electric dust-collecting unit according to a sixth exemplary embodiment of the present invention;
Fig. 12B is a sectional view showing a relation between the electrodes and a deodorizing filter of the electric dust-collecting unit according to the sixth exemplary embodiment of the present invention;
Fig. 13A is a sectional view showing a relation between electrodes and a dust-collecting filter of an electric dust-collecting unit according to a seventh exemplary embodiment of the present invention;
Fig. 13B is a sectional view showing a relation between the electrodes and a deodorizing filter of the electric dust-collecting unit according to the seventh exemplary embodiment of the present invention;
Fig. 13C is a sectional view showing a relation between the electrodes, the dust-collecting filter and the deodorizing filter of the electric dust-collecting unit according to the seventh exemplary embodiment of the present invention;
Fig. 13D is a sectional view showing a relation between the electrodes, the deodorizing filter and the dust-collecting filter of the electric dust-collecting unit according to the seventh exemplary embodiment of the present invention;
Fig. 14 is a sectional view of an electric dust-collecting unit according to an eighth exemplary embodiment of the present invention;
Fig. 15A is an exploded sectional view showing a state wherein a holder frame is being depressed, in the electric dust-collecting unit according to the eighth exemplary embodiment;
Fig. 15B is a sectional view showing another state wherein the holder frame is pressed-in, in the electric dust-collecting unit according to the eighth exemplary embodiment;
Fig. 16 is a rear view of a grid frame of the electric dust-collecting unit according to the eighth exemplary embodiment;
Fig. 17 is a partially sectioned view showing a configuration of a surrounding wall of the electric dust-collecting unit according to the eighth exemplary embodiment;
Fig. 18 is a front view of an electric dust-collecting unit according to a ninth exemplary embodiment of the present invention, with a grid frame removed;
Fig. 19 is a sectional view showing a configuration of a reinforcing rib of the electric dust-collecting unit according to the ninth exemplary embodiment;
Fig. 20A is a perspective view of a power supply unit of an electric dust-collecting unit according to a tenth exemplary embodiment of the present invention;
Fig. 20B is a perspective view of a dust collector unit of the electric dust-collecting unit according to the tenth exemplary embodiment of the present invention;
Fig. 21 is a side view of the power supply unit of the electric dust-collecting unit according to the tenth exemplary embodiment of the present invention;
Fig. 22 is a side view of the dust collector unit of the electric dust-collecting unit according to the tenth exemplary embodiment of the present invention;
Fig. 23 is a front view of the electric dust-collecting unit according to the tenth exemplary embodiment of the present invention, showing a state wherein the dust collector unit is being installed to the power supply unit;
Fig. 24 is another front view of the electric dust-collecting unit according to the tenth exemplary embodiment of the present invention;
Fig. 25 is a front view of an electric dust-collecting unit according to an eleventh exemplary embodiment of the present invention, showing a state wherein the unit is being attached;
Fig. 26 is a bottom view of the electric dust-collecting unit according to the eleventh exemplary embodiment of the present invention;
Fig. 27 is a sectional view showing a state wherein the electric dust-collecting unit is installed to radiator fins, according to the eleventh exemplary embodiment of the present invention;
Fig. 28 is a perspective view of a receptacle of the electric dust-collecting unit according to the eleventh exemplary embodiment of the present invention;
Fig. 29 is a sectional view showing a relation between a support portion and the receptacle of the electric dust-collecting unit according to the eleventh exemplary embodiment of the present invention;
Fig. 30 is a sectional view showing a state wherein the electric dust-collecting unit according to the eleventh exemplary embodiment of the present invention is installed to an air conditioner; and
Fig. 31 is a sectional view of a conventional electric dust-collecting unit.

### REFERENCE MARKS IN THE DRAWINGS

- 101, 101A, 201, 201A, 201B and 301: needle-shaped electrode
- 202: needle-shaped electrode plate
- 203 and 203B: parallel electrode
- 204, 204A, 204B, 204C 204D and 204F: perforated electrode
- 205 and 205A: pointed end
- 206: base portion
- 208A, 208B, 208C, 208D, 208E and 208F: dust-collecting filter
- 209A, 209B, 209C, 209D, 209E and 209F: deodorizing filter
- 210 and 210A: chemical agent
- 211: first dust-collecting filter
- 212: first deodorizing filter
- 213: second dust-collecting filter
- 301: needle-shaped electrode
- 302: grid frame
- 303: storage frame
- 305 and 305A: ground electrode
- 306 and 306A: first filter
- 307: second filter
- 308 and 308A: holder frame
- 309 and 418: handle
- 310: groove
- 311: hook
- 312: link hole
- 313: surrounding wall
- 314, 314A and 410: opening
- 315: surrounding rib
- 316: rib
- 317: reinforcing rib
- 318: notch
- 319: thrust rib
- 320: jut rib
- 401 and 401A: dust collector unit
- 402: dust-collector side connecting plane
- 403: raised connecting plane
- 404: ground-side receiving terminal
- 405 and 405: power supply unit
- 406: power-supply side connecting plane
- 407: recessed connecting plane
- 408: ground-side supply terminal
- 409: high voltage contact point
- 411: projecting barrier
- 412: support portion
- 413: receptacle
- 414: mounting leg
- 415: radiator fin
- 416: catch
- 417: projection

### DETAILED DESCRIPTION OF THE PREFERRED EMBODIMENTS

Referring now to the drawings, description is provided hereinafter of exemplary embodiments of the present invention.

### (FIRST EXEMPLARY EMBODIMENT)

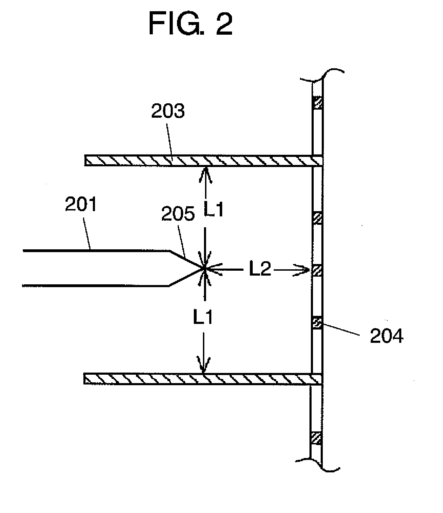

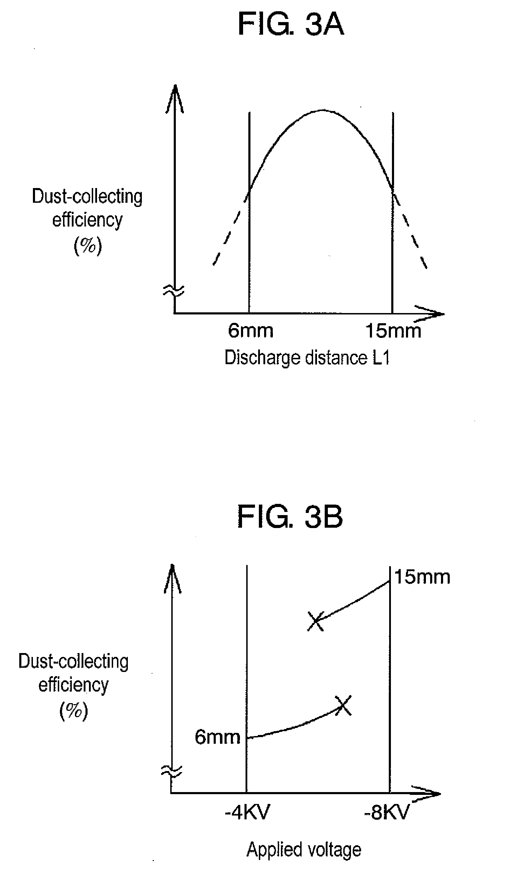

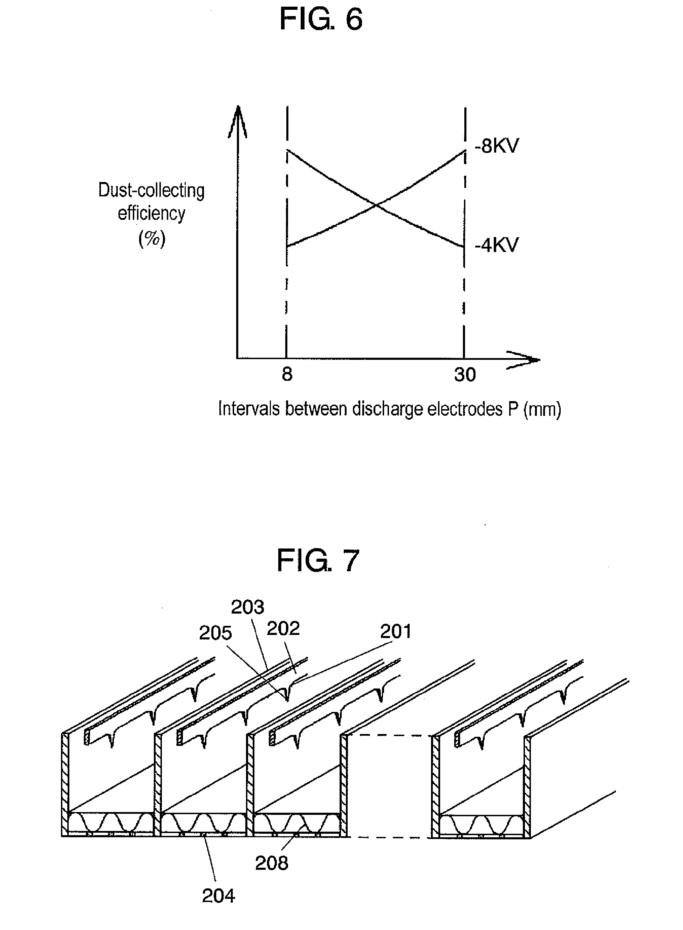

Fig. 1 to Fig. 7 depict an electric dust-collecting unit according to the first exemplary embodiment of the present invention. As shown in Fig. 1 to Fig. 7, the electric dust-collecting unit of the present invention comprises needle-shaped electrode plates 202, each having a plurality of needle-shaped electrodes 201, and disposed in parallel to one another at regular intervals. The electric dust-collecting unit also comprises parallel electrodes 203 disposed in parallel to needle-shaped electrode plates 202 at regular intervals, and tabular perforated electrode 204 supporting parallel electrodes 203. Needle-shaped electrodes 203 are so disposed that their pointed ends 205 are oriented in a direction of airflow. Perforated electrode 204 is located at the downstream side of needle-shaped electrodes 201 in a position to confront their pointed ends 205, so that parallel electrodes 203 and perforated electrode 204 are arranged into a shape of generally the letter U in a manner to surround needle-shaped electrodes 201.

The letter "U" itself consists of straight line portions and a curved line portion. By stating the shape of "the letter U", however, it does not necessarily mean that the configuration is limited to such shape in this invention. For instance, the curved line portion of the letter "U" may instead be a straight line. In other words, all lines of the letter can be configured of straight lines. Or, the straight line portions of the letter "U" may as well be curved lines. It might be more appropriate to refer the letter of such configuration as "the letter C". It should be understood, however, that the letter U is defined in the present invention as covering any such variations including the above configurations.

Here, distance L1 from pointed ends 205 of needle-shaped electrodes 201 to parallel electrodes 203 is designed to be equal to distance L2 to perforated electrode 204. That is, the structure is so composed that L1 = L2, L1 has a distance of 6mm to 15mm, an applied voltage is set to -4kV to -8kV depending on the discharge distance L1 of needle-shaped electrodes 201, and needle-shaped electrodes 201 provided on needle-shaped electrode plates 202 are formed at intervals P of 8mm to 30mm.

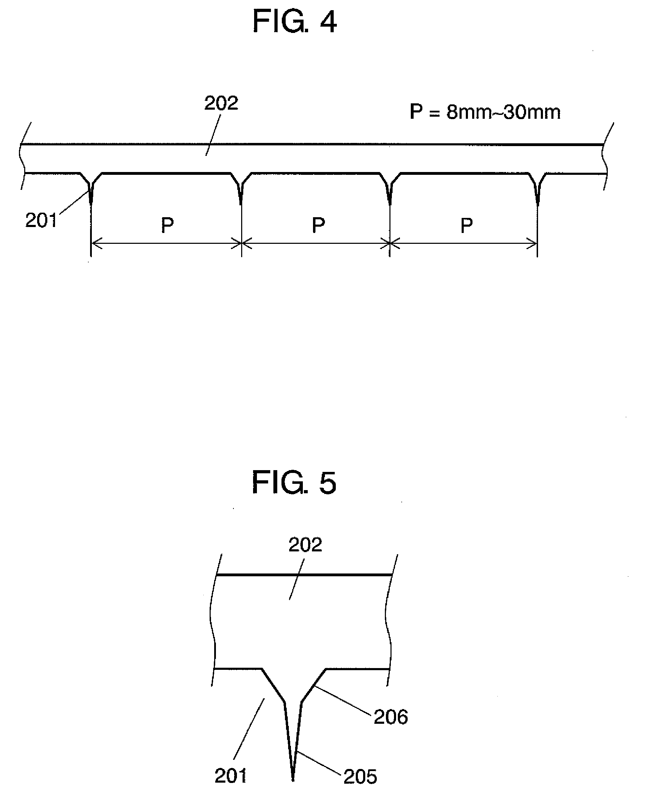

In Fig. 5, needle-shaped electrode plates 202 are fabricated of a sheet metal, and each of needle-shaped electrode 201 is configured to have pointed end 205 of an acute angle and base portion 206 having a wide portion for reinforcement of pointed end 205. Positions of pointed ends 205 of needle-shaped electrodes 201 are secured to make them steady when frame 207 is molded with a resin material which holds base portions 206.

Numbers and dimensions of needle-shaped electrode plates 202, parallel electrodes 203 and perforated electrodes 204 are adjusted according to electric dust-collecting units of various types and shapes. Dust-collecting filter 208 is disposed at the upstream side of perforated electrode 204, and deodorizing filter 209 is disposed at the downstream side.

In the above structure, an intensely high electric field is produced around needle-shaped electrodes 201 disposed in an air intake opening, by a high voltage of negative polarity impressed to needle-shaped electrodes 201 with respect to perforated electrode 204 serving as a ground electrode, when the electric dust-collecting unit mounted to a passage of air stream inside a product such as an air conditioner (not shown) with a high voltage supply device (not shown) receives a flow of air through it.

When needle-shaped electrodes 201 discharge electrons into the air, the electrons join with molecules of the air and ionize them, which in turn attach to and statically charge dust particles in the air. The statically charged dust particles are collected by parallel electrodes 203 as well as dust-collecting filter 208 disposed in the upstream side of perforated electrode 204. In addition, deodorizing filter 209 disposed in the downstream side of perforated electrode 204 collects odorous components and deodorize them.

Since parallel electrodes 203 and perforated electrode 204 are arranged into the shape of the letter U in a manner to surround needle-shaped electrodes 201, the electrical discharges can occur in many directions from pointed ends 205 of needle-shaped electrodes 201 toward parallel electrodes 203 and perforated electrode 204, or the confronting electrodes, and thereby improving the dust-collecting performance. This structure can reduce the thickness of the electric dust-collecting unit despite of the fact that it can make needle-shaped electrodes 201 disposed close to perforated electrode 204.

Moreover, since distance L1 from pointed ends 205 of needle-shaped electrodes 201 to parallel electrodes 203 is designed to be equal to distance L2 to perforated electrode 204, this arrangement can reduce variations in discharge intensity as well as directions of the electrical discharges. In addition, since parallel electrodes 203 and perforated electrode 204 are arranged to form the shape of generally the letter U, the electrical discharges occur uniformly in the spaces formed therebetween, thereby improving the dust-collecting performance.

Furthermore, the discharge distances L1 and L2 from pointed ends 205 of needle-shaped electrodes 201 to parallel electrodes 203 are set to be 6mm to 7.5mm, and the applied voltage is set to -4kV to -8kV according to needle-shaped electrodes 201, or the discharge electrodes, so as to obtain conditions of effective electrical discharges and to reduce variations in the dust-collecting efficiency.

In addition, since the intervals P of needle-shaped electrodes 201 formed on needle-shaped electrode plates 202 are made to be 8mm to 30mm to properly maintain distances of the electrical discharges between one pointed end 205 to adjoining pointed end 205 of needle-shaped electrodes 201, the structure can avoid interference between pointed ends 205 and produce the electrical discharges evenly over parallel electrodes 203 and perforated electrode 204.

Needle-shaped electrode plates 202 are fabricated of a sheet metal, and each of needle-shaped electrodes 201 is configured to have pointed end 205 of an acute angle and base portion 206 having a wide portion to reinforce pointed end 205. Since this structure secures pointed ends 205 of needle-shaped electrodes 201 in predetermined positions by rigidly fixing widened base portions 206 with the resin material when used to mold needle-shaped electrodes 201 into frame 207, it allows fabrication of narrowly spaced needle-shaped electrodes 201 with sheet metal.

Numbers and dimensions of needle-shaped electrode plates 202, parallel electrodes 203 and perforated electrodes 204 are adjusted according to electric dust-collecting units of various types and shapes. A single dust collector unit consisting of needle-shaped electrode plate 202 and perforated electrode 204 may be prepared as a basic unit, so as to make it versatile and adaptable to various types and shapes of electric dust-collecting units. In other words, this can help make needle-shaped electrode plates 202 and parallel electrodes 203 as components for common use. Perforated electrode 204 can be enlarged to any integral multiple of the basic unit by simply increasing an area size of the component. Any combination, application and expansion in the above manner enable the electric dust-collecting unit to be designed according to a configuration and dimensions of an airflow area, for example, of any variety of air conditioners, thereby achieving a cost reduction by way of the component standardization.

### (SECOND EXEMPLARY EMBODIMENT)

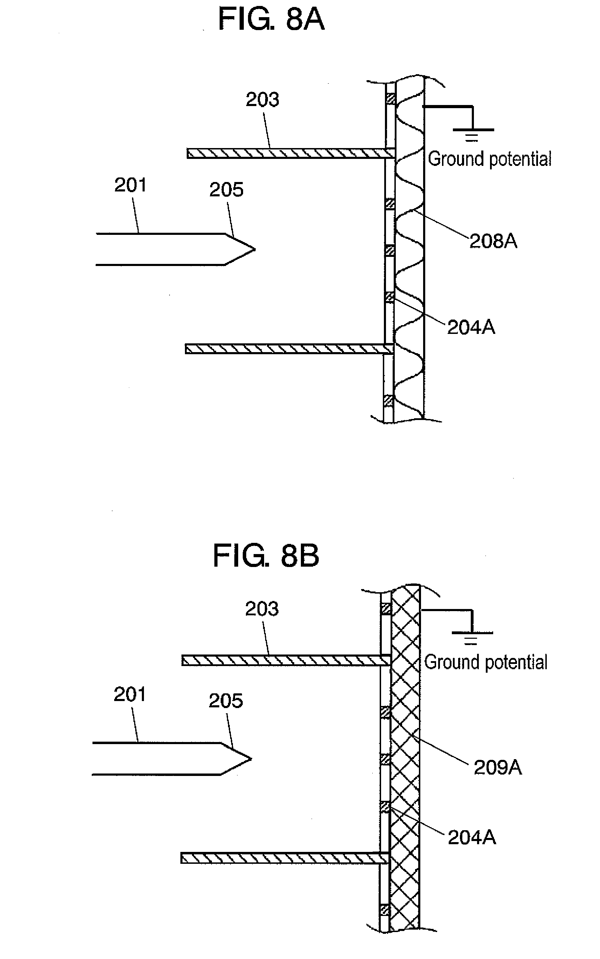

Fig. 8A and Fig. 8B show electrodes of an electric dust-collecting unit according to the second exemplary embodiment. As shown in Fig. 8A and Fig. 8B, the electric dust-collecting unit is provided with any of dust-collecting filter 208A and deodorizing filter 209A disposed at the downstream side in a manner to contact with perforated electrode 204A.

When dust-collecting filter 208A is provided at the downstream side of perforated electrode 204A in a manner to contact with it, as shown in the Fig. 8A, it can collects dust statically charged by electrical discharges generated from pointed ends 205 of needle-shaped electrodes 201 when a high voltage is applied thereto.

If deodorizing filter 209A is provided at the downstream side of perforated electrode 204A in a manner to contact with it, as shown in the Fig. 8B, it collects odorous components along with the dust statically charged by the electrical discharges generated from pointed ends 205 of needle-shaped electrodes 201 when the high voltage is applied thereto, so as to make effective use of the deodorizing function while improve the dust-collecting efficiency.

### (THIRD EXEMPLARY EMBODIMENT)

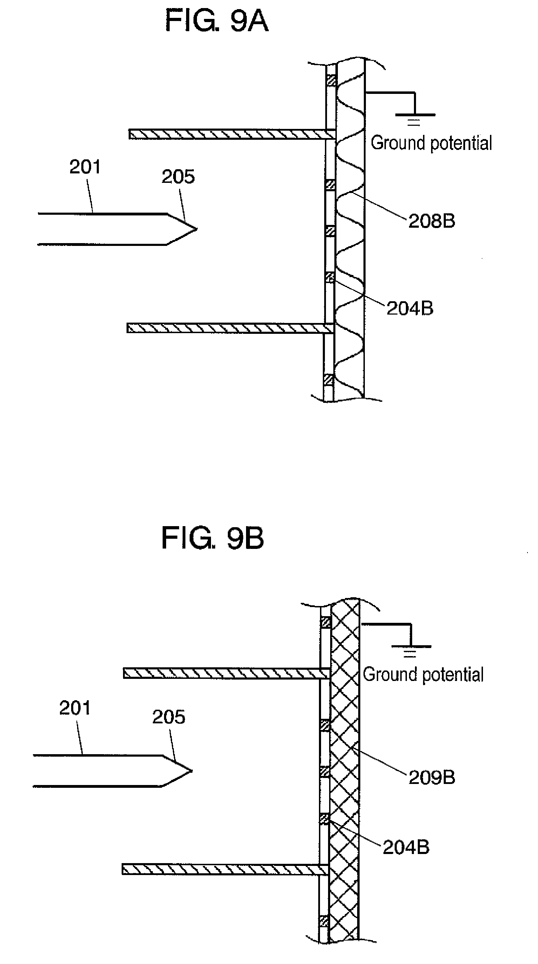

Fig. 9A and Fig. 9B show electrodes of an electric dust-collecting unit according to the third exemplary embodiment. As shown in Fig. 9A and Fig. 9B, any of dust-collecting filter 208B and deodorizing filter 209B disposed at the downstream side of perforated electrode 204B is comprised of having an electrical conductivity.

In the Fig. 9A, when dust-collecting filter 208B having an electrical conductivity is provided in a manner to contact with perforated electrode 204B, the entire surface of dust-collecting filter 208B can be maintained at the ground potential. This structure can make dust-collecting filter 208B collect dust once charged statically by electrical discharges generated from pointed ends 205 of needle-shaped electrodes 201 when a high voltage is applied thereto, and improve the dust-collecting efficiency.

In Fig. 9B, deodorizing filter 209B having an electrical conductivity is provided in a manner to contact with perforated electrode 204B. In this case, the entire surface of deodorizing filter 209B can be kept at the ground potential. This structure can make deodorizing filter 209B collect dust charged statically as well as odorous components contained in the dust when the high voltage is applied and electrical discharges are generated from pointed ends 205 of needle-shaped electrodes 201, thereby increasing the dust-collecting efficiency as well as the deodorizing efficiency.

### (FOURTH EXEMPLARY EMBODIMENT)

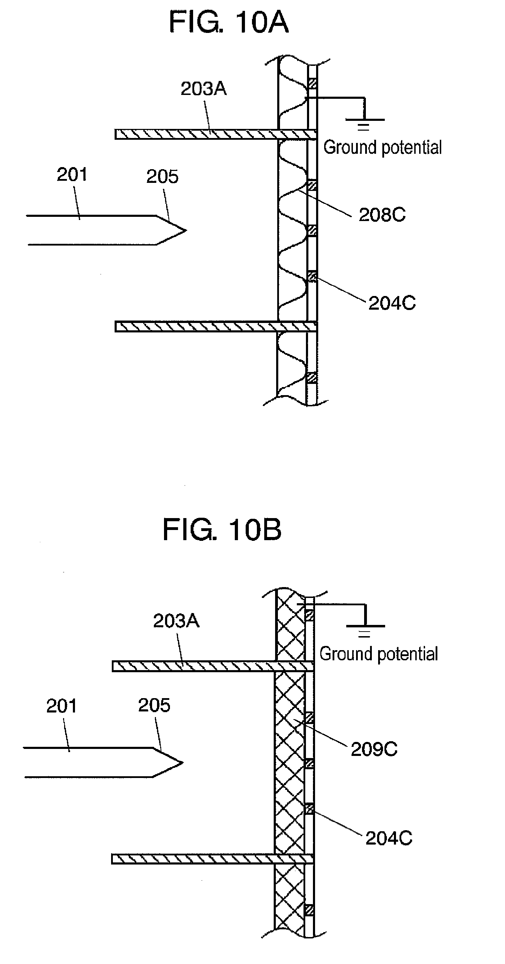

Fig. 10A and Fig. 10B show electrodes of an electric dust-collecting unit according to the fourth exemplary embodiment. As shown in Fig. 10A and Fig. 10B, a plurality of dust-collecting filters 208C or deodorizing filters 209C are disposed in parallel to one another at the upstream side of perforated electrode 204C in a manner that back surfaces of dust-collecting filters 208C or deodorizing filters 209C are kept in contact with perforated electrode 204C, and side surfaces of dust-collecting filters 208C or deodorizing filters 209C are kept in contact with parallel electrodes 203A.

In Fig. 10A, when dust-collecting filters 208C are provided at the upstream side of perforated electrode 204C, dust-collecting filters 208C can be maintained at the ground potential. This structure can make all of the parallel arranged dust-collecting filters 208B collect statically charged dust around pointed ends 205 of needle-shaped electrodes 201 to which the high voltage is applied.

In Fig. 10B, if deodorizing filters 209C are provided at the upstream side of perforated electrode 204C, deodorizing filter 209C can be maintained at the ground potential. The structure can make all of the parallel arranged deodorizing filters 209C collect statically charged dust as well as odorous components contained in the dust around pointed ends 205 of needle-shaped electrodes 201 to which the high voltage is applied.

Since these structures keep the side surfaces of dust-collecting filters 208C or deodorizing filters 209C in contact with parallel electrodes 203A, they can maintain ground continuity of the side surfaces of dust-collecting filters 208C or deodorizing filters 209C in addition to their back surfaces. The structures thus can keep the ground potential widely, and maintain the dust-collecting efficiency or the deodorizing efficiency at a level higher than a predetermined degree.

### (FIFTH EXEMPLARY EMBODIMENT)

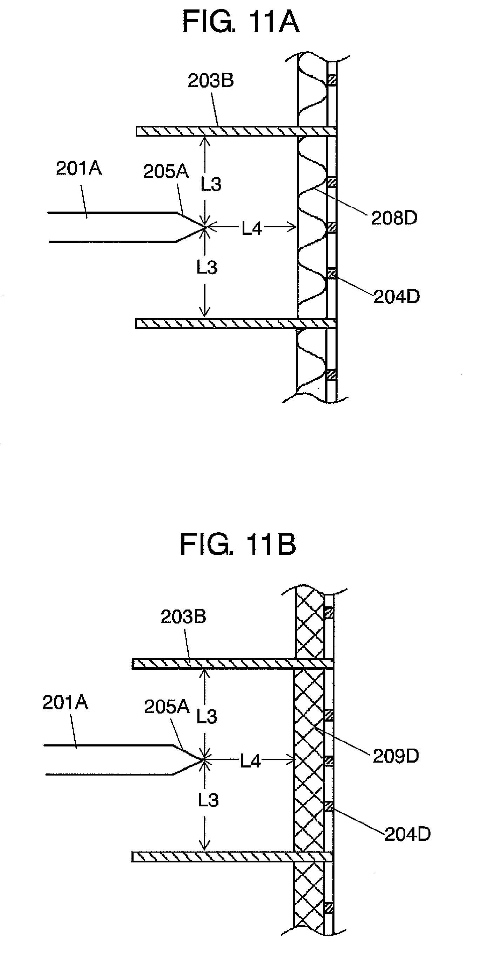

Fig. 11A and Fig. 11B show electrodes of an electric dust-collecting unit according to the fifth exemplary embodiment. As shown in Fig. 11A and Fig. 11B, dust-collecting filters 208D or deodorizing filters 209D disposed at the upstream side of perforated electrodes 204D are provided with electrical conductivity. In addition, distance L3 from pointed ends 205 of needle-shaped electrodes 201A to parallel electrodes 203B and distance L4 to perforated electrode 204D are arranged to be equal (i.e., L3 = L4).

In the structure shown in Fig. 11A, in which distance L3 is equal to distance L4, dust-collecting filters 208D is so disposed that their upper surfaces lie at the same distance from pointed ends 205A of needle-shaped electrodes 201A as distance L3 from pointed ends 205A to parallel electrodes 203B, in order to avoid a problem of having electrical discharges to concentrate on the upper surfaces of dust-collecting filters 208D. This structure facilitates the electrical discharges to spread generally evenly over dust-collecting filters 208D and parallel electrodes 203B while also expand an area of the discharges, so as to improve the dust-collecting efficiency.

Also, if distance L3 is arranged to be equal to distance L4 in the structure shown in Fig. 11B, it can avoid the problem of electrical discharges to concentrate on the upper surfaces of deodorizing filters 209D. The structure can hence promote the electrical discharges to spread generally evenly over deodorizing filters 209D and parallel electrodes 203B to collect statically charged dust and odorous components contained in the dust, and improve the dust-collecting efficiency as well as the deodorizing efficiency.

### (SIXTH EXEMPLARY EMBODIMENT)

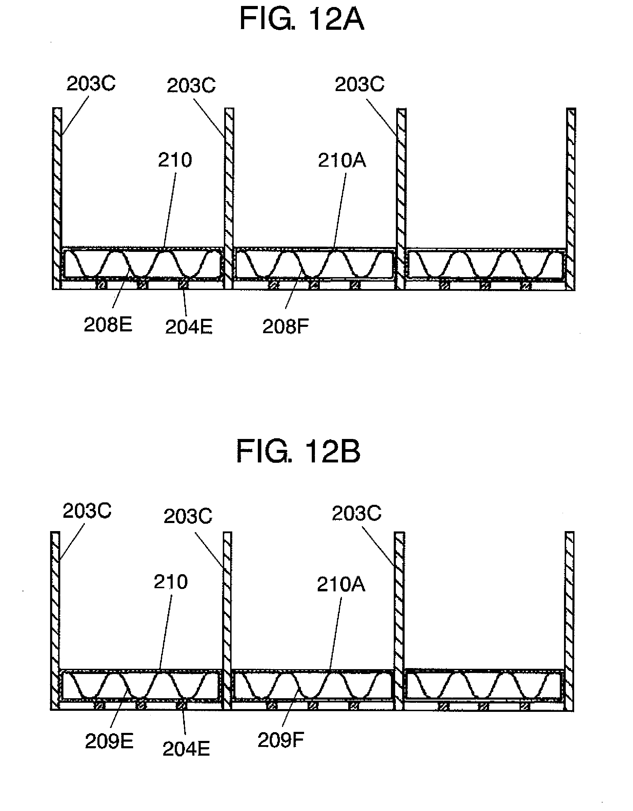

Fig. 12A and Fig. 12B show electrodes used in an electric dust-collecting unit according to the sixth exemplary embodiment of the present invention.

As shown in Fig. 12A and Fig. 12B, a plurality of chemical agents 210 and 210A, which are not easily applicable in a form of multiple layer, are coated separately on each of two dust-collecting filters 208E and 208F, or deodorizing filters 209E and 209F provided in individual sections separated by parallel electrodes 203C.

In Fig. 12A, dust-collecting filter 208E coated with chemical agent 210 and another dust-collecting filter 208F coated with chemical agent 210A are disposed in parallel to each other across parallel electrode 203C, at the upstream side of perforated electrode 204E. According to this structure, both dust-collecting filters 208E and 208F collect dust. Since each of dust-collecting filters 208E and 208F are coated individually with different chemical agents 210 and 210A, they can inactivate or decompose a plurality of different kinds of harmful substances. For instance, chemical agent 210 inactivates such detrimental organism as viruses, and another chemical agent 210A inactivates or decomposes a different harmful substance such as allergen.

In Fig. 12B, deodorizing filter 209E coated with chemical agent 210 and another deodorizing filter 209F coated with chemical agent 210A are disposed in parallel to each other across parallel electrode 203C, at the upstream side of perforated electrode 204E. Deodorizing filters 209E and 209F collect dust and odorous components contained in the dust. Since each of deodorizing filters 209E and 209F are coated individually with different chemical agents 210 and 210A, chemical agent 210, for instance, can inactivates or decomposes harmful gases including the odorous components and chemical agent 210A inactivates or decomposes detrimental organism such as various germs.

In this sixth exemplary embodiment, although the description was provided by showing two sets of dust-collecting filters 208E and 208F or deodorizing filters 209E and 209F, there can be more filters than just two for coating other kinds of chemical agents without causing any change in the activeness and effectiveness.

### (SEVENTH EXEMPLARY EMBODIMENT)

Fig. 13A to Fig. 13D show electrodes together with needle-shaped electrode 201B as used in an electric dust-collecting unit according to the seventh exemplary embodiment of the present invention. As shown in Fig. 13A to Fig. 13D, either first dust-collecting filter 211 or first deodorizing filter 212 is disposed at the upstream side of perforated electrode 204F, and second dust-collecting filter 213 or second deodorizing filter 214 is disposed at the downstream side of perforated electrode 204F. This structure is so designed as to make adjustment possible in number of any of first dust-collecting filters 211 and first deodorizing filters 212 as well as dimensions of any of second dust-collecting filter 213 and second deodorizing filter 214.

In Fig. 13A, first dust-collecting filter 211 is disposed at the upstream side and second dust-collecting filter 213 is disposed at the downstream side respectively of perforated electrode 204F. Both first dust-collecting filter 211 and second dust-collecting filter 213 collect statically charged dust in the vicinity of needle-shaped electrode 201B.

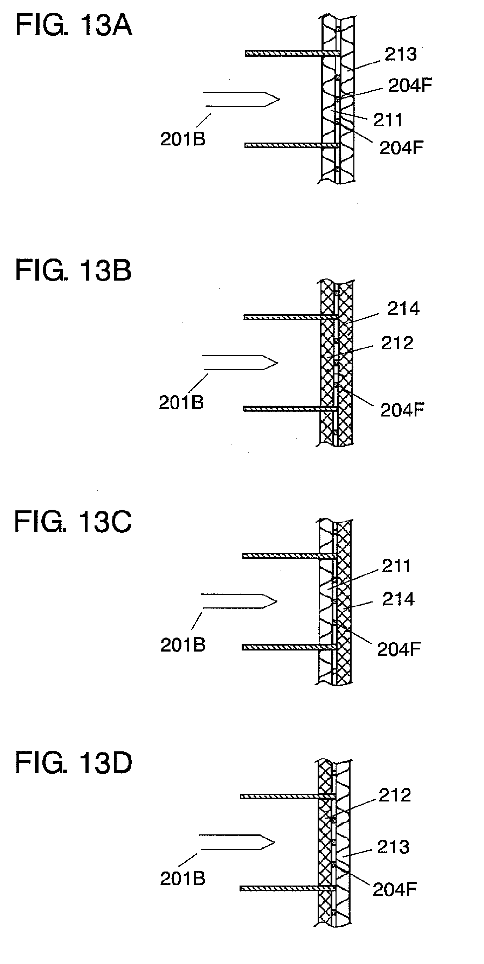

In Fig. 13B, first deodorizing filter 212 is disposed at the upstream side and second deodorizing filter 214 is disposed at the downstream side respectively of perforated electrode 204F. This structure mainly collects odorous components contained in the dust.

In Fig. 13C, first dust-collecting filter 211 is disposed at the upstream side and second deodorizing filter 214 is disposed at the downstream side respectively of perforated electrode 204F. First dust-collecting filter 211 collects dust and second deodorizing filter 214 collects odorous components contained in the dust.

In Fig. 13D, first deodorizing filter 212 is disposed at the upstream side and second dust-collecting filter 213 is disposed at the downstream side respectively of perforated electrode 204F. First deodorizing filter 212 collects odorous components contained in dust and second dust-collecting filter 213 collects dust.

In the structures of Fig. 13A and Fig. 13C, the electric dust-collecting unit can be adapted to any shape and dimensions of a space available for installation in an air conditioner by increasing a number of identically configured first dust-collecting filters 211 disposed at the upstream side of perforated electrode 204F. It is also possible to adapt the electric dust-collecting unit according to an increase or decrease in the space available for installation when an alteration of perforated electrode 204F is needed due to a size of the air conditioner, by adjusting dimensions of any of second dust-collecting filter 213 and second deodorizing filter 214. These structures can facilitate common use and standardization of the components, and make design changes easily.

### (EIGHTH EXEMPLARY EMBODIMENT)

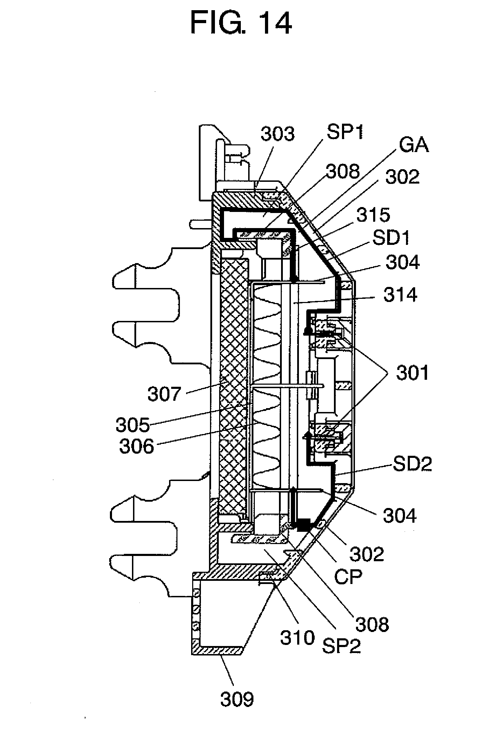

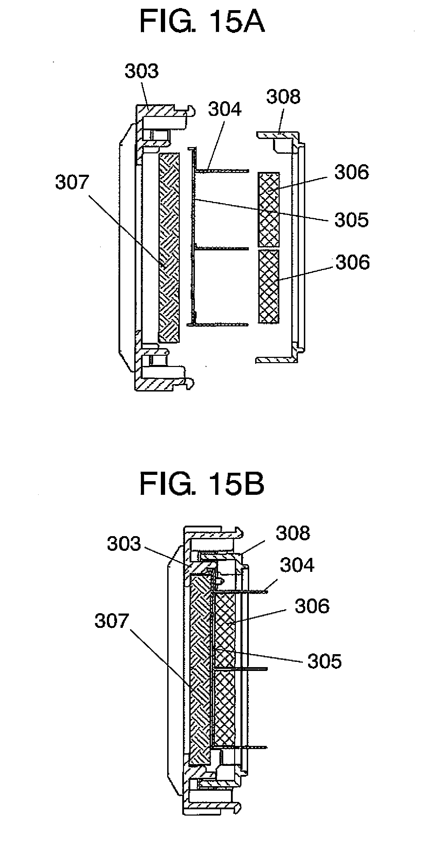

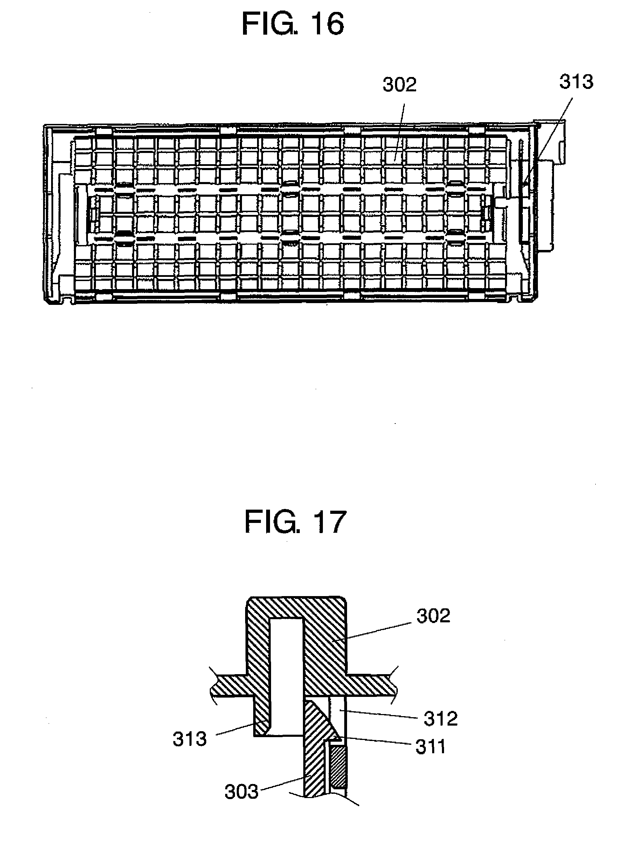

Fig. 14, Fig. 15A, Fig. 15B, Fig. 16 and Fig. 17 show an electric dust-collecting unit according to the eighth exemplary embodiment. The electric dust-collecting unit comprises grid frame 302 for fixing a plurality of needle-shaped electrodes 301, storage frame 303 to which grid frame 302 is mounted, and ground electrode 305 provided with parallel electrodes 304 of a configuration storable in storage frame 303. First filter 306 having a dust-collecting function is disposed at the upstream side of ground electrode 305, and second filter 307 having a deodorizing function is disposed at the downstream side of ground electrode 305. Holder frame 308 for fixing ground electrode 305 bearing first filter 306 and second filter 307 is placed in storage frame 303 in a manner so that grid frame 302 does not come into contact with holder frame 308. That is, holder frame 308 and grid frame 302 are positioned with a predetermined space between them. Storage frame 303 is provided with groove 310 in a base portion of handle 309 formed on it, so that an edge of grid frame 302 can fit into groove 310 in order to couple grid frame 302 with storage frame 303.

Storage frame 303 is also provided with hook 311 at its edge, while grid frame 302 is provided with link hole 312 for hook 311 to engage therewith. Grid frame 302 has surrounding wall 313 which encircles link hole 312. Holder frame 308 has opening 314 formed in it for air to pass through, and surrounding rib 315 in a shape to encircle the entire perimeter of opening 314.

In the above structure, an intensely high electric field is produced around needle-shaped electrodes 301 disposed to grid frame 302 serving an air intake opening, by a high voltage of negative polarity impressed to needle-shaped electrodes 301 with respect to ground electrode 305, when the electric dust-collecting unit mounted to a passage of air stream inside a product such as an air conditioner (not shown) with a high voltage supply device (not shown) receives a flow of air through it.

When free electrons attach to dust particles, they statically charge the dust particles. The charged dust particles do not collide to one another, and they are collected by first filter 306 disposed at the upstream side of ground electrode 305 while odorous components are collected and the air deodorized by second filter 307 disposed at the downstream side of ground electrode 305.

Referring to Fig. 14, there are provided storage frame 303, ground electrode 305, holder frame 308, and grid frame 302 fixing needle-shaped electrodes 301, as has been described previously. Although grid frame 302 is coupled with storage frame 303, it is one of the features of the eighth exemplary embodiment that grid frame 302 is so constructed as not to come into contact with holder frame 308. While this structure provides a thin-profile configuration allowing grid frames 302 to closely couple with storage frame 303, it also ensures through-the-air spacing and creepage distances of predetermined extents.

Fig. 14 schematically shows spaces SP1 and SP2 as well as creepage distances SD1 and SD2. The creepage distance SD1 shown here is a condition that a comparatively large (i.e., long) creepage distance can be maintained under the structure in which grid frame 302 and holder frame 308 are not allowed to contact with each other. It can be seen that creepage distance SD1 is not obstructed even in a relatively narrow space GA where grid frame 302 and holder frame 308 confront each other, and it further extends to a part of parallel electrode 304 through space SP1.

It is also known, on the other hand, that creepage distance SD2 becomes shorter as compared to creepage distance SD1 when grid frame 302 and holder frame 308 are made to contact with each other since blockade CP is formed in a portion of the contact. In other words, it shows that creepage distance SD2 extends only to a part of flat electrode 304 immediately after passage through blockade CP instead of extending toward space SP2. Blockade CP discussed here does not actually exist in this eighth exemplary embodiment, but it is shown only for the purpose of making the above explanation.

The electric dust-collecting unit according to this eighth exemplary embodiment has groove 310 formed in the base portion of handle 309 provided on storage frame 303 so that the edge of grid frame 302 is fitted into groove 310. Groove 310 formed in storage frame 303 plays the role of a guide when grid frame 302 is assembled to storage frame 303.

Since grid frame 302 has link hole 312 for engagement with hook 311 provided at the edge of storage frame 303, surrounding wall 313 is formed on grid frame 302 to encircle link hole 312, so as to avert the problem of dust form being allowed to enter through link hole 312 acting as an air passage into the dust collecting space where electrical contacts are present, and to prevent a leakage current from occurring inside the dust collecting space.

In addition, surrounding rib 315 is formed to encircle the entire perimeter of opening 314 provided in holder frame 308 for air to pass through. Holder frame 308 can make both positioning and fixing simultaneously of ground electrode 305 on which second filter 307 and first filter 306 are attached. Surrounding rib 315 provided to encircle the entire perimeter of opening 314 can improve a physical strength of holder frame 308, and maintain the predetermined extent of creepage distances.

### (NINTH EXEMPLARY EMBODIMENT)

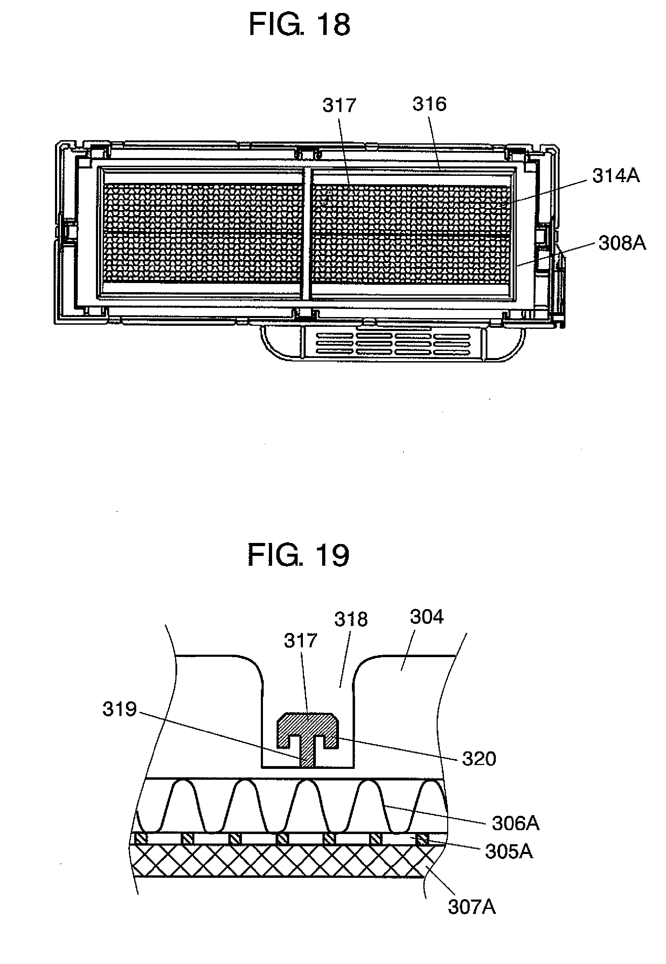

Fig. 18 and Fig. 19 relate to the ninth exemplary embodiment, as they show an electric dust-collecting unit with its grid frame removed. This electric dust-collecting unit is provided with reinforcing rib 317 bridging between a pair of ribs 316 in the lengthwise direction to form opening 314A in holder frame 308A. It is also provided with notch 318 formed by cutting off a portion of ground electrode 305A up to the close vicinity of an upper surface of first filter 306A. Reinforcing rib 317 is constructed into such a shape in cross section as having thrust rib 319 protruding vertically, and jut ribs 320 projecting both sides from the top of thrust rib 319 and extending vertically, so that the bottom edge of reinforcing rib 317 abuts to the upper edge of notch 318.

In the above structure, one reinforcing rib 317 or a plurality of them are provided across opening 314A of holder frame 308A so as to prevent ground electrode 305A carrying first filter 306A and second filter 307 A from shifting upward, thereby obviating abnormal electrical discharges. Reinforcing rib 317 is composed of thrust rib 319 and jut ribs 320 in the cross-sectional shape. This structure reinforces holder frame 308A which secures ground electrode 305A carrying first filter 306A and second filter 307A while also ensures the sufficient creepage distances with jut ribs 320, so as to prevent leakage from taking place.

### (TENTH EXEMPLARY EMBODIMENT)

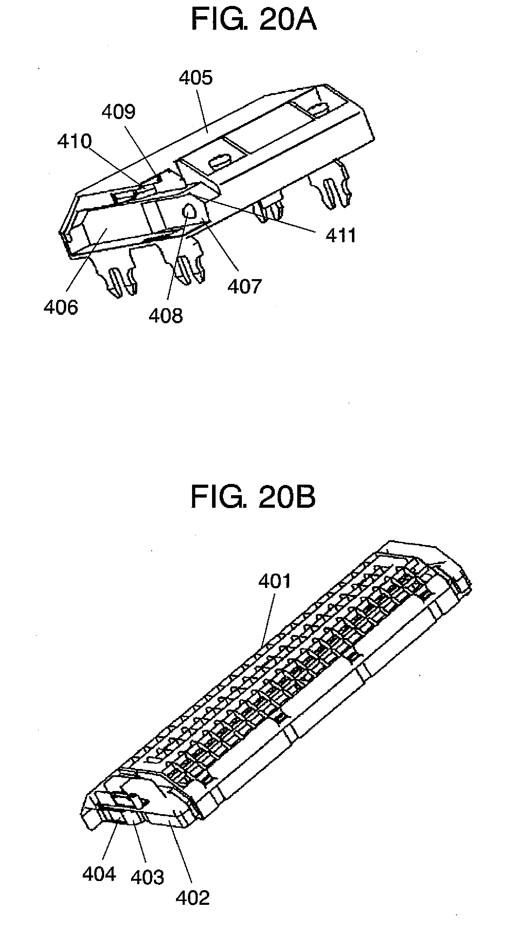

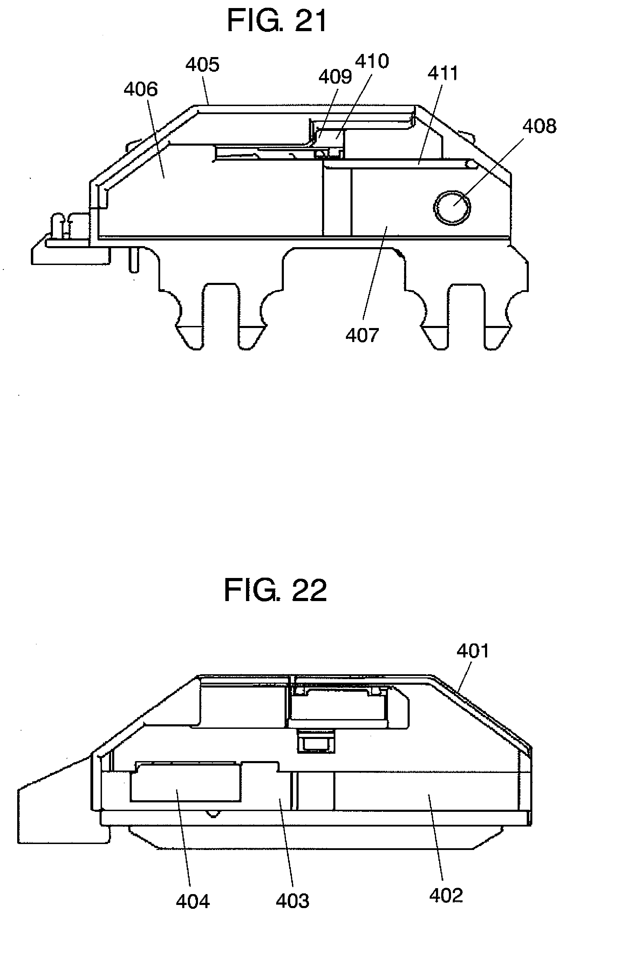

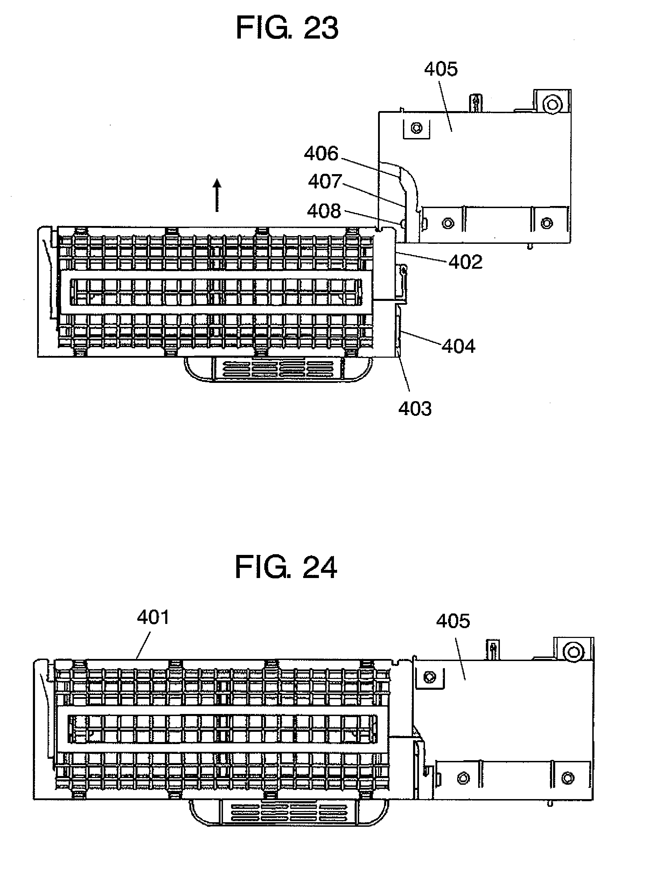

Fig. 20A to Fig. 24 relate to the tenth exemplary embodiment of the present invention, and they show a power supply unit attached to an electric dust-collecting unit. The tenth exemplary embodiment will also be illustrated as an example embodied to provide the electric dust-collecting unit adaptable for improvement of dust-collecting performance and reduction of the thickness.

As shown in Fig. 20A to Fig. 24, the electric dust-collecting unit comprises dust-collector side connecting plane 402 and raised connecting plane 403 provided on an end surface of dust collector unit 401, and ground-side receiving terminal 404 disposed on raised connecting plane 403. It also comprises power-supply side connecting plane 406 and recessed connecting plane 407 provided on an end surface of power supply unit 405, and ground-side supply terminal 408 disposed on recessed connecting plane 407. Ground-side receiving terminal 404 and ground-side supply terminal 408 are connected when raised connecting plane 403 of dust collector unit 401 and recessed connecting plane 407 of power supply unit 405 are engaged together.

Moreover, the electric dust-collecting unit is provided with recessed portion 410 in which high-voltage contact point 409 is positioned in power-supply side connecting plane 406 of power supply unit 405, and projecting barrier 411 formed on power-supply side connecting plane 406 of power supply unit 405. Projecting barrier 411 is formed on recessed connecting plane 407 provided in power-supply side connecting plane 406 of power supply unit 405 into a height up to power-supply side connecting plane 406 and in a position between high-voltage contact point 409 and ground-side supply terminal 408, which serves as a grounding contact point.

In the above structure, dust-collector side connecting plane 302 of dust collector unit 401 is inserted into engagement with power-supply side connecting plane 406 provided on power supply unit 405 when dust collector unit 401 is attached to power supply unit 405. Dust collector unit 401 is retained when its raised connecting plane 403 engages with recessed connecting plane 407 of power supply unit 405. Ground-side receiving terminal 404 disposed on raised connecting plane 403 of dust collector unit 401 then comes into continuity to ground-side supply terminal 408 disposed on recessed connecting plane 407 of power supply unit 405. High-voltage contact point 409 disposed on power supply unit 405 also comes into continuity to a high-voltage receiving point (not shown) provided on dust collector unit 401, and dust collector unit 401 is completely attached to power supply unit 405.

Both dust collector unit 401 and power supply unit 405 are provided with raised connecting plane 403 and recessed connecting plane 407 in their respective end surfaces for engagement. This configuration decreases an area of contact, and reduces a frictional resistance during removal and replacement of dust collector unit 401 from and to power supply unit 405, so as to make it unnecessary to function the fastening part utilizing elasticity of ground-side receiving terminal 404 and ground-side supply terminal 408 until immediately before their complete engagement. It can thus improve the manipulability, ensure sufficiently large extents of the creepage distances and through-the-air clearances between connecting terminals of the high-voltage side and the low-voltage side, and achieve a reduction in thickness of the electric dust-collecting unit.

This structure has projecting barrier 411 formed on power supply unit 405 in the position between high-voltage contact point 409 and ground-side supply terminal 408. They ensure sufficiently large extent of the creepage distances between high-voltage contact point 409 and ground-side supply terminal 408.

Projecting barrier 411 is formed on recessed connecting plane 407 of power supply unit 405 where ground-side supply terminal 408 is provided. Projecting barrier 411 serves as a guide for sliding dust collector unit 401 during removal and replacement, and improves the usability since projecting barrier 411 is formed into a height equivalent to that of power-supply side connecting plane 406. As has been described, use of the raised connecting plane and the recessed connecting plane can ease removal and replacement of the components for supplying the high voltage, and thereby the electric dust-collecting unit is adaptable for such application as air cleaners, which require removal and replacement of ionizing units and dust-collecting units to which high voltages are applied.

### (ELEVENTH EXEMPLARY EMBODIMENT)

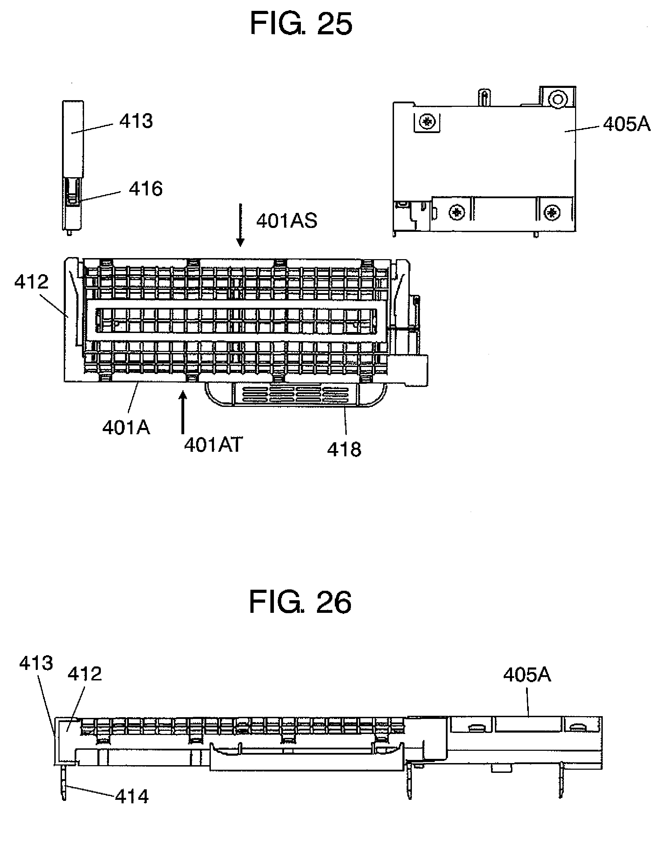

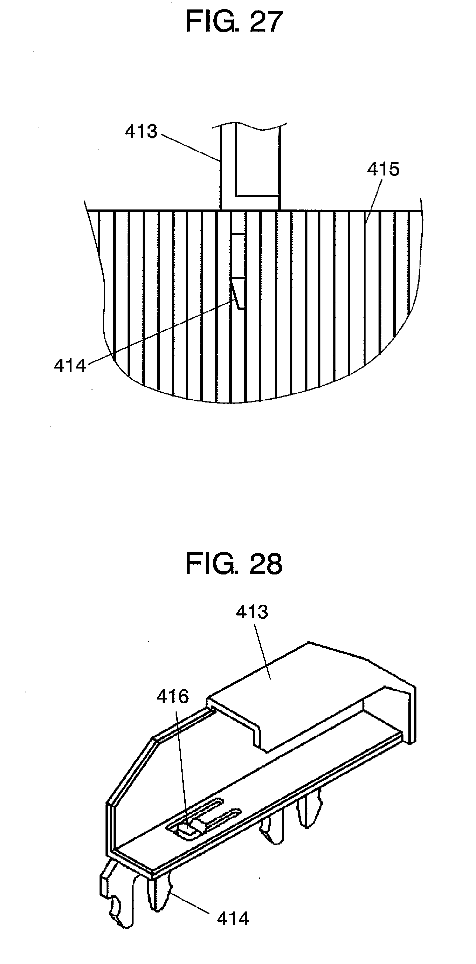

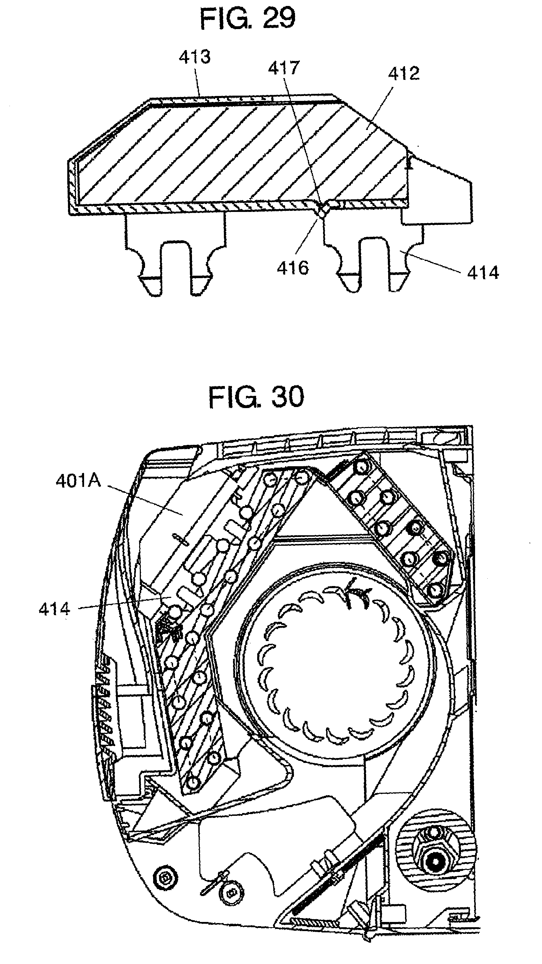

Fig. 25 to Fig. 30 illustrate an electric dust-collecting unit according to the eleventh exemplary embodiment 11, showing a state wherein the unit is being attached. Power supply unit 405A is attached to one side 401AS of dust collector unit 401A. Support portion 412 is provided on an edge at the other side 401AT of dust collector unit 401A, where power supply unit 405A is not attached. In addition, receptacle 413 is provided for accepting and retaining support portion 412, which is so configured as to be mountable to radiator fins 415 of an air conditioner by way of mounting legs 414. Receptacle 413 is provided with catch 416 in its bottom surface, so as to have catch 416 hold projection 417 formed on support portion 412. Power supply unit 405A is also provided with mounting legs 414.

Handle 418 formed on dust collector unit 401A is positioned closer to the one side where power supply unit 405A is attached thereto.

In the above structure, power supply unit 405A and receptacle 413 are first attached to radiator fins 415. The electric dust-collecting unit is mounted thereafter to radiator fins 415 of an air conditioner. When mounting dust collector unit 401A, one side of it is attached to power supply unit 405A in a manner that has been described in the tenth exemplary embodiment. The other side 401AT of dust collector unit 401A, where power supply unit 405A is not attached, has support portion 412 formed on it. Support portion 412 formed on the one end of dust collector unit 401A is inserted into receptacle 413 mounted to radiator fins 415. Both ends of dust collector unit 401A are retained when projection 417 formed on support portion 412 engages with catch 416 provided in receptacle 413.

The structure discussed here averts a trouble that dust collector unit 401A is mounted in a tilted position. Since this maintains the air passage vertical, it avoids reduction in performance of the electric dust-collecting unit, and simplifies the structure for installation of the electric dust-collecting unit.

Furthermore, the structure can ensure reliable retention of the both ends of dust collector unit 401A by way of engaging projection 417 formed on support portion 412 with catch 416 provided in receptacle 413, and distribute a resistance evenly between right and left during removal and replacement, so as to substantially improve the manipulability.

Moreover, handle 418 on dust collector unit 401A is positioned closer to the one end where it is engaged to power supply unit 405A. Since handle 418 is provided at the side closer to power supply unit 405A, where the resistance against removal and replacement of dust collector unit 401A is larger, it can ease the removal and replacement of dust collector unit 401A and improve the manipulability.

### INDUSTRIAL APPLICABILITY

The electric dust-collecting unit of the present invention is adaptable for such application as air cleaners, negative ion generators and the like products by taking advantages of the structure comprising the parallel electrodes supported by the perforated electrode, which has a high potential for reduction in the thickness, as discussed above. In addition, since use of the raised connecting plane and the recessed connecting plane helps ease removal and replacement of components supplying high voltages, the invention is adaptable for use in air cleaners, which require removal and replacement of ionizing units and dust-collecting units carrying application of high voltages, and it thus has a high industrial applicability.

## Claims

1. An electric dust-collecting unit comprising:
a plurality of needle-shaped electrodes, each having a pointed end oriented in a direction of airflow;
parallel electrodes disposed in parallel to the needle-shaped electrodes; and
a perforated electrode disposed at a downstream side of the needle-shaped electrodes in a position confronting the pointed ends,
wherein the parallel electrodes and the perforated electrode are arranged in a shape of a letter U in a manner to surround the needle-shaped electrodes.

2. The electric dust-collecting unit of claim 1, wherein a distance from the pointed ends of the needle-shaped electrodes to the parallel electrodes is made to be equal to a distance from the pointed ends to the perforated electrode.

3. The electric dust-collecting unit of one of claim 1 and claim 2,
wherein a distance of electrical discharges from the pointed ends of the needle-shaped electrodes to the parallel electrodes is set to a range of 6mm to 15mm, and a voltage applied to the electrodes for discharges to a range of -4kV to -8kV.

4. The electric dust-collecting unit of one of claim 1 and claim 2,
wherein the needle-shaped electrodes provided on the needle-shaped electrode plate are at intervals of 8mm to 30mm.

5. The electric dust-collecting unit of one of claim 1 and claim 2,
wherein the needle-shaped electrode plate is fabricated of a sheet metal, and the needle-shaped electrodes are each formed to have a pointed end of an acute angle and a base portion widened to reinforce the pointed end.

6. The electric dust-collecting unit of claim 1, further comprising:
needle-shaped electrode plates, each having the plurality of needle-shaped electrodes, and disposed in parallel to one another at regular intervals;
parallel electrodes disposed in parallel to the needle-shaped electrode plates at regular intervals; and
a tabular perforated electrode supporting the parallel electrodes,
wherein the electric dust-collecting unit is adaptable to an increase or decrease in an area available for installation by adjusting numbers of the needle-shaped electrode plates and the parallel electrodes as well as dimensions of the perforated electrode.

7. The electric dust-collecting unit of one of claim 1 and claim 2, further comprising a dust-collecting filter disposed at the downstream side of the perforated electrode in a manner to contact therewith.

8. The electric dust-collecting unit of one of claim 1 and claim 2, further comprising a deodorizing filter disposed at the downstream side of the perforated electrode in a manner to contact therewith.

9. The electric dust-collecting unit of claim 7, wherein the dust-collecting filter disposed at the downstream side of the perforated electrode is provided with an electrical conductivity.

10. The electric dust-collecting unit of claim 8, wherein the deodorizing filter disposed at the downstream side of the perforated electrode is provided with an electrical conductivity.

11. The electric dust-collecting unit of claim 1, further comprising any of dust-collecting filters and deodorizing filters disposed in parallel to one another at a upstream side of the perforated electrode, and at least back surfaces of the dust-collecting filters or the deodorizing filters are kept in contact with the perforated electrode.

12. The electric dust-collecting unit of claim 11, wherein any of the dust-collecting filters and the deodorizing filters disposed at the upstream side of the perforated electrode are made to be electrically conductive, and the dust-collecting filters or the deodorizing filters are so disposed that a distance of their upper surfaces from the pointed ends of the needle-shaped electrodes is equal to a distance from the pointed ends to the parallel electrodes.

13. The electric dust-collecting unit of claim 11, further comprising a plurality of chemical agents coated separately on each of at least two dust-collecting filters or deodorizing filters.

14. The electric dust-collecting unit of claim 1, further comprising any of first dust-collecting filters and first deodorizing filters disposed at a upstream side of the perforated electrode, and any of a second dust-collecting filter and a second deodorizing filter at the downstream side of the perforated electrode.

15. The electric dust-collecting unit of claim 14, wherein a number of any of the first dust-collecting filters and the first deodorizing filters disposed at the upstream side of the perforated electrode as well as dimensions of any of the second dust-collecting filter and the second deodorizing filter disposed at the downstream side of the perforated electrode are adjusted, thereby composing the electric dust-collecting unit adaptable to an increase or decrease in an area available for installation.

16. An electric dust-collecting unit comprising:
a storage frame storing a ground electrode provided with a first filter and a second filter;
a holder frame securing the second filter and the ground electrode; and
a grid frame fixing a needle-shaped electrode,
wherein the grid frame is coupled to the storage frame.

17. The electric dust-collecting unit of claim 16, wherein the storage frame has a groove formed in a base portion of a handle provided thereon in a manner so that an edge of the grid frame is fitted into the groove.

18. The electric dust-collecting unit of claim 16, wherein the storage frame has a hook formed at one edge thereof, and the grid frame has a link hole for engaging with the hook and a surrounding wall formed thereon in a manner to encircle the link hole.

19. The electric dust-collecting unit of claim 16, wherein the holder frame has an opening formed therein for airflow and a surrounding rib encircling an entire perimeter of the opening.

20. The electric dust-collecting unit of claim 16, wherein the holder frame has a reinforcing rib bridging between a pair of ribs in the lengthwise direction forming an opening provided therein, the ground electrode has a notch formed by cutting off a portion thereof up to a close vicinity of an upper surface of the first filter, and further wherein a bottom edge of the reinforcing rib abuts to an upper edge of the notch.

21. The electric dust-collecting unit of claim 20, wherein the reinforcing rib has a shape in cross section comprising a thrust rib protruding vertically, and jut ribs projecting both sides from a top of the thrust rib and extending vertically.

22. A electric dust-collecting unit comprising:
a dust-collector side connecting plane and a raised connecting plane provided on an end surface of a dust collector unit;
a ground-side receiving terminal disposed on the raised connecting plane;
a power-supply side connecting plane and a recessed connecting plane provided on an end surface of a power supply unit; and
a ground-side supply terminal disposed on the recessed connecting plane,
wherein the ground-side receiving terminal and the ground-side supply terminal are connected when the raised connecting plane of the dust collector unit and the recessed connecting plane of the power supply unit are engaged with each other.

23. The electric dust-collecting unit of claim 22, wherein the power supply unit has a recessed portion in the power-supply side connecting plane wherein a high-voltage contact point is positioned, and a projecting barrier formed on the power-supply side connecting plane in a position between the high-voltage contact point and a grounding contact point.

24. The electric dust-collecting unit of claim 23, wherein the projecting barrier is raised up to a height of the power-supply side connecting plane of the power supply unit.

25. The electric dust-collecting unit of claim 22, wherein the dust collector unit is attached to one side of the dust collector unit, a support portion is provided on the other side of the dust collector unit, the electric dust-collecting unit further comprises a receptacle for accepting and retaining the support portion, and the receptacle is mountable to radiator fins of an air conditioner.

26. The electric dust-collecting unit of claim 25, wherein the receptacle has a catch provided in a bottom surface thereof for engaging with a projection formed on the support portion.

27. The electric dust-collecting unit of claim 22, wherein the dust collector unit is provided with a handle on a position close to one side where the power supply unit is attached.
